# FASCICULE DE BREVET EUROPEEN

(11) **EP 0 948 527 B1**
(45) Date de publication et mention de la délivrance du brevet: **05.06.2002**
(21) Numéro de dépôt: 97952998.9
(22) Date de dépôt: 23.12.1997
(51) Int. Cl.: C07K 7/64, A61K 38/13

(54) **DERIVES DE CYCLOSPORINE, LEUR PREPARATION ET LES COMPOSITIONS PHARMACEUTIQUES QUI LES CONTIENNENT**
CYCLOSPORINDERIVATE, DEREN HERSTELLUNG UND PHARMAZEUTISCHE ZUSAMMENSETZUNGEN
CYCLOSPORIN DERIVATIVES, THEIR PREPARATION AND PHARMACEUTICAL COMPOSITIONS CONTAINING THEM

(30) Priorité: 24.12.1996 FR 9615956
(43) Date de publication de la demande: 13.10.1999
(73) Titulaire: Aventis Pharma S.A., 92160 Antony (FR)
(72) Inventeur: BARRIERE, Jean-Claude, F-91400 Bures sur Yvette (FR); BASHIARDES, Georges, F-94320 Thiais (FR); CARRY, Jean-Christophe, F-92190 Meudon (FR); EVERS, Michel, F-94510 La Queue en Brie (FR); FILOCHE, Bruno, F-94000 Créteil (FR); LECONTE, Jean-Pierre, F-91800 Brunoy (FR); MIGNANI, Serge, F-92290 Châtenay Malabry (FR)
(86) Numéro de dépôt international: FR9702405
(87) Numéro de publication internationale: WO9828329

(56) Documents cités:
- EP-A- 0 194 972
- EP-A- 0 484 281
- US-A- 4 814 323
- BILLICH A ET AL: "MODE OF ACTION OF SDZ NIM 811, A NONIMMUNOSUPPRESSIVE CYCLOSPORIN A ANALOG WITH ACTIVITY AGAINST HUMAN IMMUNODEFICIENCY VIRUS (HIV) TYPE 1: INTERFERENCE WITH HIV PROTEIN-CYCLOPHILIN A INTERACTIONS" JOURNAL OF VIROLOGY, vol. 69, no. 4, avril 1995, pages 2451-2461, XP002022423

## Description

La présente invention concerne des dérivés de cyclosporine de formule générale : 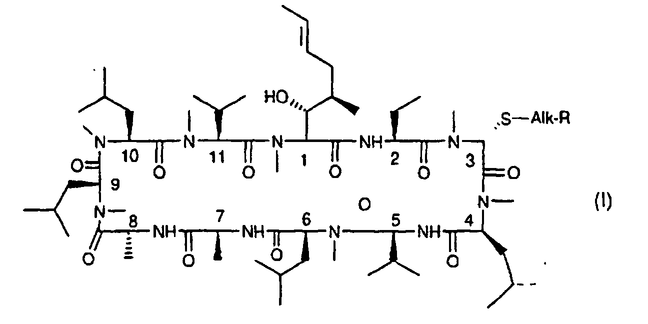 leurs sels, leur préparation et les compositions pharmaceutiques qui les contiennent.

Ces dérivés sont utiles pour le traitement et/ou la prophylaxie des infections à rétrovirus, et plus particulièrement du SIDA (syndrome d'immuno.déficience acquise) et de syndromes associés [ARC (AIDS related complex)]. Ils présentent l'avantage d'être très faiblement immunosuppresseurs.

Des dérivés de cyclosporine modifiés en position -3 ont été précédemment décrits comme agents immunosuppresseurs, dans le brevet européen EP 194972.

Des dérivés [4'-hydroxy-Meleu]⁴ cyclosporine ont été décrits dans le brevet européen EP 484 281. Ces dérivés sont utiles pour le traitement du SIDA et ne sont pas immunosuppresseurs.

Il a été trouvé maintenant que les dérivés de la cyclosporine de formule générale (I) dans laquelle :
- Alk représente un radical alcoylène contenant 2 à 6 atomes de carbone en chaîne droite ou ramifiée ou cycloalcoyléne contenant 3 à 6 atomes de carbone et
- R représente
   . soit un radical carboxy ou alcoyloxycarbonyle,
   . soit un radical -NR₁R₂ pour lequel R₁ et R₂ identiques ou différents représentent des atomes d'hydrogène, ou des radicaux alcoyle, alcènyle (2 à 4C), cycloalcoyle (3 à 6C), phényle éventuellement substitué (par un atome d'halogène, alcoyloxy, alcoyloxycarbonyle, amino, alcoylamino ou dialcoylamino). ou représentent des radicaux benzyle ou hétérocyclyle saturé ou insaturé contenant 5 ou 6 chaînons et 1 à 3 hétéroatomes, ou pour lequel R₁ et R₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alcoyle, phényle ou benzyle,
   . soit un radical de formule générale : 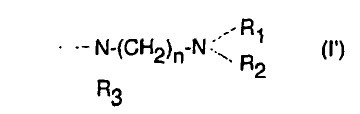 pour lequel R₁ et R₂ sont définis comme ci-dessus, R₃ représente un atome d'hydrogène ou un radical alcoyle et n est un nombre entier de 2 à 4,
les portions ou radicaux alcoyle définis ci-dessus étant droits ou ramifiés et contenant 1 à 4 atomes de carbone,
ainsi que leurs sels pharmaceutiquement acceptables lorsqu'ils existent, sont particulièrement intéressants du fait de leur activité puissante et de leur caractère très faiblement immunosuppresseur.

Dans la formule générale (I), lorsque R₁ et/ou R₂ représentent hétérocyclyle, celui-ci peut être avantageusement choisi parmi pyridyle, tétrahydropyridyle, pipéridyle, imidazolyle, oxazolyle, thiazolyle.

Lorsque R₁ et R₂ forment hétérocyclyle avec l'atome d'azote auquel ils sont attachés, à titre d'exemple, le radical hétérocyclyle peut être choisi parmi azétidinyle, pipéridyle, pipérazinyle, N-méthyl pipérazinyle, N-phényl pipérazinyle, N-benzyl pipérazinyle, pyridyle, imidazolyle, morpholino, thiomorpholino, tétrahydropyridyle, méthyl tétrahydropyridyle (par exemple 4-méthyl tétrahydropyridyle), phényl tétrahydropyridyle (par exemple 4-phényl tétrahydropyridyle).

Selon la présente invention les produits de formule générale (I) peuvent être obtenus par action d'un disulfure de formule générale :

R-Alk-S-S-Alk-R (II)

dans laquelle R et Alk sont définis comme ci-dessus, et dont le cas échéant les fonctions pouvant interférer avec la réaction ont été préalablement protégées, sur une forme activée de la cyclosporine A, puis élimine le cas échéant le radical protecteur.

On entend par forme activée de la cyclosporine A, une forme activée sur la sarcosine en position 3. Cette forme activée de la cyclosporine A est préparée de préférence in situ. L'activation s'effectue généralement sous atmosphère inerte, par traitement par un dérivé organométallique (lithien notamment, comme le n-butyllithium, le diisopropyl amidure de lithium ou un mélange par exemple). Il est également possible de préparer la forme activée de la cyclosporine A dans l'ammoniac liquide en présence d'un amidure alcalin (sodium, lithium par exemple), à une température comprise entre -32 et -38°C, dans un éther (notamment tétrahydrofurane, t.butyléthyléther ou un mélange).

L'addition du disulfure de formule générale (II), s'effectue avantageusement dans un solvant organique tel qu'un hydrocarbure (hexane par exemple) ou un éther (diéthyléther, tétrahydrofurane ou t.butylméthyléther par exemple) à une température comprise entre -78 et 0°C. Il est parfois préférable d'opérer sous azote.

Lorsque les substituants du radical R peuvent interférer avec la réaction, il est préférable de les protéger préalablement par des radicaux compatibles et pouvant être mis en place et éliminés sans toucher au reste de la molécule. De plus le radical hydroxy présent sur la cyclosporine peut être éventuellement protégé par tout groupement qui n'interfère pas avec la réaction.

A titre d'exemple les groupements protecteurs peuvent être choisis parmi les radicaux décrits par T.W. GREENE, Protective Groups in Organic Synthesis, J. Wiley-Interscience Publication (1991) ou par Mc Omie, Protective Groups in Organic Chemistry, Plenum Press (1973).

Le disulfure de formule générale (II) peut être préparé selon ou par analogie avec les méthodes décrites dans les exemples.

Les nouveaux dérivés de la cyclosporine de formule générale (I) peuvent être purifiés le cas échéant par des méthodes physiques telles que la cristallisation ou la chromatographie.

Les dérivés de la cyclosporine selon l'invention pour lesquels R est carboxy peuvent être transformés en sels métalliques ou en sels d'addition avec une base azotée selon les méthodes connues en soi. Ces sels peuvent être obtenus par action d'une base métallique (par exemple alcaline ou alcalino terreuse), de l'ammoniaque ou d'une amine sur un produit selon l'invention, dans un solvant approprié tel que l'eau ou un alcool. Le sel formé précipite après concentration éventuelle de la solution ; il est séparé par filtration.

Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropyl-amine, N,N-diméthyléthanolamine, benzylamine, dicyclohexylamine, N-benzylphénéthylamine, N,N'-dibenzyléthylènediamine, diphénylènediamine, benzhydrylamine, quinine, choline, arginine, lysine, leucine, dibenzylamine).

Les dérivés de la cyclosporine selon l'invention pour lesquels R est NR₁R₂ peuvent être transformés en sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, p.toluènesulfonates, iséthionates, embonates ou avec des dérivés de substitution de ces composés).

Les nouveaux dérivés de cyclosporine selon la présente invention sont particulièrement utiles pour la prophylaxie et le traitement des affections à rétrovirus et plus particulièrement du SIDA et de syndromes associés. Par prophylaxie on sous-entend notamment le traitement des sujets qui ont été exposés aux virus VIHs, en particulier les séropositifs asymptomatiques qui présentent le risque de développer la maladie dans les mois ou les années à venir après la primoinfection.

Les produits selon l'invention manifestent une activité anti-rétrovirus à des concentrations dépourvues d'effet cytotoxique ou cytostatique.

L'activité des produits de formule générale (I) a été mise en évidence dans les techniques décrites par Pauwells et Coll., J. Virol. Meth., 20, 309 (1988) et par O. Schwatz et Coll., AIDS Research and Human Retroviruses, 4(6), 441-48 (1988) et citées par J.F. Mayaux et al. Proc. Nat. Acad. Sci. USA, 91, 3564-68 (1994). Dans ces techniques les produits selon l'invention se sont montrés actifs à des concentrations de 3 à 350 nM (IC₅₀).

D'un intérêt particulier sont les produits de formule générale (I) pour lesquels :
- Alk représente un radical alcoyléne contenant 2 à 6 atomes de carbone en chaîne droite ou ramifiée et
- R représente un radical -NR₁R₂ pour lequel R₁ et R₂ identiques ou différents représentent des atomes d'hydrogène, ou des radicaux alcoyle, alcènyle (2 à 4C), phényle éventuellement substitué (par un atome d'halogène, alcoyloxy, alcoyloxycarbonyle, amino, alcoylamino ou dialcoylamino), ou représentent des radicaux benzyle ou pour lequel R₁ et R₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alcoyle,
les portions ou radicaux alcoyle définis ci-dessus étant droits ou ramifiés et contenant 1 à 4 atomes de carbone, ainsi que leurs sels pharmaceutiquement acceptables lorsqu'ils existent ;

Selon un aspect encore plus préférentiel de l'invention, les dérivés de cyclosporine de formule générale (I) pour lesquels :
- Alk représente un radical alcoyiène contenant 2 à 5 atomes de carbone en chaîne droite ou ramifiée et
- R représente un radical -NR₁R₂ pour lequel R₁ et R₂ identiques ou différents représentent des atomes d'hydrogène, ou des radicaux alcoyle, allyle, phényle ou benzyle ou pour lequel R₁ et R₂ forment avec l'atome d'azote auquel ils sont rattachés un hétérocycle choisi parmi azétidinyle, pipéridyle, pipérazinyle, N-méthyl pipérazinyle, N-phényl pipérazinyle, N-benzyl pipérazinyle, imidazolyle, morpholino, tétrahydropyridyle, méthyl tétrahydropyridyle, phényl tétrahydropyridyle.
les portions ou radicaux alcoyle définis ci-dessus étant droits ou ramifiés et contenant 1 à 4 atomes de carbone, ainsi que leurs sels pharmaceutiquement acceptables lorsqu'ils existent ;

Et parmi ces produits notamment les dérivés de cyclosporine ci-après :
- [(R)-2-(N,N-diéthylamino)éthylthio-Sar]³-cyclosporine A ;
- [(R)-2-(N,N-diméthylamino)éthylthio-Sar]³-cyclosporine A ;
- [(R)-2-(1-pipéridyl)-éthylthio-Sar]³ cyclosporine A ;
- [(R)-2-(N-méthyl-N-i.propylamino)éthylthio-Sar]³ cyclosporine A ;
- [(R)-2-(N-méthyl-N-t.butylamino)éthylthio-Sar]³ cyclosporine A ;
ainsi que leurs sels pharmaceutiquement acceptables lorsqu'ils existent.

Les exemples suivants donnés à titre non limitatif illustrent la présente invention.

### Exemple 1

Le méthane sulfonate de la [(R)-2-(N,N-diéthylamino)éthylthio-Sar]³-cyclosporine A, est préparé selon la méthode suivante :

A une solution de 3,53 cm³ de diisopropylamine (préalablement distillée sur hydrure de calcium) dans 45 cm³ de tétrahydrofuranne (préalablement distillé sur sodium), refroidie à une température voisine de -10°C et sous azote, on ajoute en 20 minutes, 15,6 cm³ d'une solution de n-butyllithium 1,6 M dans l'hexane en maintenant la température à 0°C. Le mélange est agité à 0°C pendant 20 minutes, puis il est refroidi à une température voisine de -78°C. La solution ainsi obtenue est transférée, sous azote, par une canne de transfert, sur une solution de 2 g de cyclosporine A dans 40 cm³ de tétrahydrofuranne préalablement refroidie à une température voisine de -78°C en maintenant la température vers -75°C. Le mélange résultant est agité à -75°C pendant 10 minutes, puis 6,3 cm³ d'une solution de n-butyllithium 1,6 M dans l'hexane sont ajoutés en 4 minutes. L'agitation est maintenue durant 20 minutes puis 8,8 g de disulfure de di-[2-(N,N-diéthylamino)éthyle] sont ajoutés en 2 minutes en maintenant la température vers -75°C. Le mélange est agité à une température voisine de -75°C pendant 30 minutes puis à 0°C pendant 18h. On verse sur le mélange réactionnel un mélange de 50 cm³ d'eau distillée glacée et d'acide chlorhydrique aqueux à 36% pour obtenir un pH voisin de 7, puis le mélange est décanté, la phase aqueuse lavée par 30 cm³ d'éther diéthylique. Les extraits organiques sont réunis, lavés avec 50 cm³ de solution saturée en chlorure de sodium, séchés sur sulfate de sodium, filtrés et concentrés sous pression réduite (2.7 kPa) à une température voisine de 40°C. Le solide obtenu est purifié par 2 chromatographies flash successives sur colonne de silice (0,04-0,063 mm) (éluant méthanol-eau-dichlorométhane 14:2:84 en volumes puis dichlorométhane-méthanol 93:7 en volumes), en recueillant des fractions de 10 cm³. Les fractions contenant le produit attendu sont réunies et concentrées sous pression réduite (2,7 kPa) à une température voisine de 40°C pour donner 0,245 g de [(R)-2-(N,N-diéthylamino)éthylthio-Sar]³ cyclosporine A sous la forme d'un solide blanc.

Dans un ballon on place sous agitation 0,245 g de [(R)-2-(N,N-diéthylamino)éthylthio-Sar]³-cyclosporine A en solution dans 15 cm³ d'éther diéthylique. Après filtration, on ajoute, en 30 secondes, 15 mg d'acide méthanesulfonique en solution dans 2 cm³ d'éther diéthylique. Le précipité blanc obtenu est filtré, lavé par 3 fois 2 cm³ d'éther diéthylique puis par 2 cm³ de pentane. On obtient ainsi 0,067 g de méthane sulfonate de [(R)-2(N,N-diéthylamino)éthylthio-Sar]³ cyclosporine A sous la forme d'un solide blanc fondant à 155°C (déc.).

Spectre de R.M.N. 1 H (400 MHz, CDCl₃, δ en ppm) ; 1,24 (d, J = 7 Hz, 3H : CH₃ 8β) ; 1,31 (d, J = 7,5 Hz, 3H : CH₃ 7β) ; 1,31 (t, J = 7 Hz, 6H : CH₃ de l'éthyle du 2-diéthylaminoéthylthio en 3α) ; 1,60 (d, J = 5 Hz, 3H : CH₃ en 1γ) ; 2,38 (mt, 1H : CH 5β) ; 2,66 - 2,75 -3,08 - 3,10 - 3,22 - 3,41 et 3,46 (7 s; respectivement 6H - 3H - 3H - 3H - 3H - 3H et 3H : 7 NCH₃ et CH₃ du méthanesulfonate) ; de 2,90 à 3,35 (mf, 8H : SCH₂CH₂N du 2-diéthylaminoéthylthio en 3α et N CH₂ de l'éthyle du 2-diéthylaminoéthylthio en 3α) ; 3,76 (mt, 1H : CH 1β) ; 4,48 (mt, 1H : CH 7α) ; 4,64 (t, J = 9 Hz, 1H : CH 5α) ; 4,81 (mt, 1H : CH 8α) ; de 4,95 à 5,05 (mt, 2H : CH 2 α et CH α d'une leucine) ; 5,06 (d, J = 11 Hz, 1H : CH 11α) ; 5,18 (dd, J = 12 et 4Hz, 1H : CH α d'une leucine) ; de 5,20 à 5,35 (mt, 2H : CH=CH) ; 5,41 (d, J = 6 Hz, 1H : CH 1α) ; 5,67 (dd, J = 10 et 4 Hz, 1H : CH α d'une leucine) ; 5,90 (s, 1H : CH 3α) ; 7,17 (d, J = 9 Hz, 1H : CONH en 5) ; 7,39 (d, J = 8 Hz, 1H : CONH en 8) ; 7,68 (d, J = 7,5 Hz, 1H : CONH en 7) ; 8,04 (d, J = 9,5 Hz, 1H : CONH en 2).

Le disulfure de di-[2-(N,N-éthylamino)éthyle] peut être préparé selon la méthode décrite dans H. Gilman J. Am. Chem. Soc. (1945) 67, 1846

### Exemple 2

La [(R)-2-(N,N-diméthylamino)éthylthio-Sar]³-cyclosporine A est préparée par selon la méthode suivante:

A une solution de 26,5 cm³ de diisopropylamine (préalablement distillée sur hydrure de calcium) dans 300 cm³ de tétrahydrofuranne (préalablement distillé sur sodium) refroidie à une température voisine de -5°C et sous azote, on ajoute en 30 minutes, 117 cm³ d'une solution de n-butyllithium 1,6 M dans l'hexane en maintenant la température à 0°C. Le mélange est agité à 0°C pendant 20 minutes, puis il est refroidi à une température voisine de -78°C. La solution ainsi obtenue est transférée,sous azote, par une canne de transfert, sur une solution de 15 g de cyclosporine A dans 270 cm³ de tétrahydrofuranne préalablement refroidie à une température voisine de -76°C en maintenant la température vers -70°C. Le mélange résultant est agité à -78°C pendant 10 minutes, puis 47 cm³ d'une solution de n-butyllithium 1,6 M dans l'hexane sont ajoutés en 10 minutes. L'agitation est poursuivie 5 minutes puis 52 g de disulfure de di-[2-(N,N-diméthylamino)éthyle] sont ajoutés lentement en maintenant la température vers -75°C. Le mélange est agité à une température voisine de -78°C pendant 30 minutes puis à 0°C pendant 18h. On verse sur le mélange réactionnel maintenu sous agitation à - 20°C, 120 cm³ d'eau distillée glacée additionnée de 80 cm³ d'acide chlorhydrique 12N de façon à obtenir un pH voisin de 7 puis le mélange est décanté et la phase aqueuse lavée par 100 cm³ d'acétate d'éthyle. Les extraits organiques sont réunis, lavés avec une solution saturée en chlorure de sodium, séchés sur sulfate de sodium, filtrés et concentrés sous pression réduite (2,7 kPa) à une température voisine de 40°C. Le solide obtenu est mis en solution dans 200 cm³ de toluène et 1000 cm³ d'eau distillée additionnée d'acide chlorhydrique 12N pour amener le pH à 2. Les phases sont décantées. La phase aqueuse est relavée par 100 cm³ de toluène et les phases toluéniques rassemblées. Celles-ci sont lavées par 200 cm³ d'eau distillée, acidifiée à pH voisin de 3. Les phases aqueuses sont réunies, additionnées de 200 cm³ de toluène puis neutralisées par une solution aqueuse de bicarbonate de sodium. La phase organique est décantée, la phase aqueuse lavée par 100 cm³ de toluène. Les phases organiques sont rassemblées, séchées sur sulfate de sodium, filtrées et concentrées sous pression réduite (2,7 kPa) à une température voisine de 40°C pour donner un solide qui est purifié par chromatographie flash sur colonne de silice (0,04-0,063 mm) (éluant dichlorométhane-méthanol 93,5:6,5 en volumes) et en recueillant des fractions de 35 cm³. Les fractions contenant le produit attendu sont réunies et concentrées sous pression réduite (2,7 kPa) à une température voisine de 40°C pour donner un solide qui est concrété dans 30 cm³ de pentane. Après filtration, on obtient 1.94 g de [(R)-2-(N,N-diméthylamino)éthylthio-Sar]³ cyclosporine A sous forme d'un solide blanc fondant vers 140°C.

Spectre de R.M.N. ¹ H (400 MHz, CDCl₃, δ en ppm) : 1,28 (d, J = 7 Hz, 3H : CH₃ 8β) ; 1,37 (d, J = 7,5 Hz, 3H : CH₃ 7β) ; 1,63 (mt, 3H : CH₃ en 1γ) ; 2,25 (s, 6H : N(CH₃)₂ du 2-diméthylaminoéthylthio en 3α) ; 2,40 (mt, 1H : CH 5β) ; de 2,50 à 2,80 (mf, 4H : SCH₂CH₂N du 2-diméthylaminoéthylthio en 3α) ; 2,71 - 3,13 - 3,14 - 3,28 - 3,46 et 3,52 (6 s; respectivement 6H - 3H - 3H - 3H - 3H et 3H : 7 NCH₃) ; 3,65 (d, J = 6 Hz, 1H : OH en 1β) ; 3,78 (mt, 1H : CH 1β) ; 4,56 (mt, 1H : CH 7α) ; 4,67 (t, J = 9 Hz, 1H : CH 5α) ; 4,85 (mt, 1H : CH 8α) ; 4,99 (dd, J = 9 et 6 Hz, 1H : CH α d'une leucine) ; de 5,00 à 5,15 (mt, 2H : CH 2α et CH α d'une leucine) ; 5,15 (d, J = 11 Hz, 1H : CH 11α) ; 5,25 (dd, J = 12 et 4 Hz, 1H : CH α d'une leucine) ; de 5,30 à 5,45 (mt, 2H : CH=CH) ; 5,51 (d, J = 6 Hz, 1H : CH 1α) ; 5,72 (dd, J = 10,5 et 4 Hz, 1H : CH α d'une leucine) ; 6,02 (s, 1H : CH 3α) ; 7,18 (d, J = 8 Hz, 1H : CONH en 8) ; 7,35 (d, J = 9 Hz, 1H : CONH en 5) ; 7,68 (d, J = 7, 5 Hz, 1H : CONH en 7) ; 7,96 (d, J = 9,5 Hz, 1H : CONH en 2).

Le sel de méthane sulfonate de la [(R)-2-(N,N-diméthylamino)éthylthio-Sar]³ cyclosporine A, est préparé de la manière suivante: dans un ballon on place sous agitation 360 mg de [(R)-2-(N,N-diméthylamino)éthylthio-Sar]³ cyclosporine A en solution dans 6 cm³ d'éther diéthylique puis on ajoute en 30 secondes 24 mg d'acide méthanesulfonique en solution dans 1 cm³ d'éther diéthylique. Le précipité blanc obtenu est filtré, lavé par 3 fois 2 cm³ d'éther diéthylique puis par 5 cm³ de pentane. On obtient ainsi après séchage à 50°C sous pression réduite (40 kPa), 0,328 mg de méthane sulfonate de [(R)-2-(N,N-diméthylamino)éthylthio-Sar]³ cyclosporine A sous la forme d'un solide blanc fondant à 155°C( déc.).

Spectre de R.M.N. ¹ H (400 MHz, (CD₃)₂SO d6, δ en ppm) : 1,21 (d, J = 7.5 Hz, 3H : CH₃ 8β) ; 1,29 (d, J = 7,5 Hz, 3H : CH₃ 7β) ; 1,69 (d, J = 6,5 Hz, 3H : CH₃ en 1γ) ; 1,99 (mt, 1H : CH 5β) ; 2,35 (s, 3H : CH₃ du méthanesulfonate) ; de 2,45 à 2,70 (mt, 2H : SCH₂ du 2-diméthylaminoéthylthio en 3α) ; 2,64 - 2,80 - 2,86 - 2,93 - 2,99 et 3,17 (6 s, respectivement 3H - 6H - 9H - 3H - 3H et 3H : 7 NCH₃ et NCH₃ du 2-diméthylaminoéthylthio en 3α) ; de 3,25 à 3,40 (mf, 2H : CH₂N du 2-diméthylaminoéthylthio en 3α) ; 3,99 (mt. 1H : CH 1β) ; 4,15 (mt, 1H : CH 7α) ; 4,26 (t, J = 9 Hz, 1H : CH 5α) ; 4,42 (s large, 1H : OH en 1β) ; 4,79 (mt, 1H : CH 8α) ; 4,89 (mt, 1H : CH 2α) ; de 5,00 à 5,15 (mt, 1H : CH α d'une leucine) ; 5,11 (d, J = 11 Hz, 1H : CH 11α) ; 5,23 (mt, 2H : CH 1α et CH α d'une leucine) ; 5,33 (dd, J = 10 et 5 Hz, 1H : CH α d'une leucine) ; de 5,30 à 5,50 et 5,62 (2 mts, 1H chacun: CH=CH) ; 5,48 (dd, J = 11 et 5 Hz, 1H : CH α d'une leucine) ; 6,87 (s, 1H : CH 3α) ; 7,64 (d, J = 7,5 Hz, 1H : CONH en 7) ; 8,24 (d, J = 9,5 Hz, 1H : CONH en 2) ; 8,28 (d, J = 8 Hz, 1H : CONH en 8) ; 8,68 (d, J = 9 Hz, 1H : CONH en 5) ; 9,28 (mf, 1H : SO₃H du méthanesulfonate).

### Exemple 3

La [(R)-2-(1-pipéridyl)-éthylthio-Sar]³ cyclosporine A est préparée selon la méthode suivante:

A une solution de 7,0 cm³ de diisopropylamine dans 40 cm³ de tétrahydrofuranne refroidie à une température voisine de 0°C et sous argon, on ajoute au goutte à goutte 31 cm³ d'une solution de n-butyllithium 1,6 M dans l'hexane. Le mélange est agité à 0°C pendant 20 minutes, puis il est refroidi à une température voisine de -78°C et une solution de 4,0 g de cyclosporine A et de 6,0 cm³ de 1,3-diméthyl-3,4,5,6-tétrahydro-2(1H)-pyrimidinone dans 20 cm³ de tétrahydrofuranne sous argon préalablement refroidie à une température voisine de -78°C est ajoutée au goutte à goutte. Le mélange résultant est agité à une température voisine de -40°C pendant 20 minutes. puis à une température voisine -78°C pendant 1h, puis on ajoute une solution de 13,8 g de disulfure de di-[2-(1-pipéridyl)éthyle] dans 20 cm³ de tétrahydrofurane au goutte à goutte. Le mélange est ensuite agité à une température voisine de -78°C pendant 10 minutes, puis il est laissé se réchauffer à une température voisine de 20°C. Il est alors traité avec 100 cm³ d'eau. La phase aqueuse est extraite 3 fois avec 100 cm³ d'acétate d'éthyle puis les extraits organiques sont réunis, lavés 4 fois avec 50 cm³ d'eau, séchés sur sulfate de magnésium et concentrés sous pression réduite (2,7 kPa) à une température voisine de 40°C. L'huile obtenue est chromatographiée sur une colonne contenant 1,6 kg de silice (0,02-0,05 mm) éluée avec de l'acétate d'éthyle à pression atmosphérique et en recueillant des fractions de 75 cm³. Les fractions contenant le produit attendu sont réunies et concentrées sous pression réduite (2,7 kPa) à une température voisine de 40°C. Le résidu est dissout dans 20 cm³ d'acétate d'éthyle, filtré sur papier, puis reconcentré dans les mêmes conditions. Le résidu obtenu est repris dans 20 cm³ d'éther éthylique, reconcentré de la même façon, puis séché à poids constant. On obtient ainsi 0,4 g de [(R)-2-(1-pipéridyl)éthylthio-Sar]³ cyclosporine A sous forme d'un solide jaune paille fondant à 132°C.

Spectre de R.M.N. ¹ H (400 MHz, CDCl₃, δ en ppm) : 1,30 (d, J = 7 Hz, 3H : CH₃ 8β) ; 1,37 (d, J = 7,5 Hz, 3H : CH₃ 7β) ; 1,65 (d, J = 5 Hz, 3H : CH₃ en 1γ) ; de 1,90 à 3,10 (mt, 14H : CH₂ de SCH₂CH₂Npipéridine en 3α) ; 2,47 (mt, 1H : CH 5β) ; 2,70 - 2,72 - 3,13 - 3,18 - 3,27 - 3,45 et 3,52 (7 s, 3H chacun : 7 NCH₃) ; 3,77 (mt, 1H : CH 1β) ; 4,55 (mt, 1H : CH 7α) ; 4,65 (t, J = 9 Hz, 1H : CH 5α) ; 4,86 (mt, 1H : CH 8α) 4,99 (dd, J = 9 et 6 Hz, 1H : CH α d'une leucine) ; de 5,00 à 5,15 (mt, 2H : CH 2α et CH α d'une leucine) ; 5,15 (d, J = 11 Hz, 1H : CH 11α) ; 5,24 (dd, J = 12 et 4 Hz, 1H : CH α d'une leucine) ; de. 5,25 à 5,45 (mt, 2H : CH=CH) ; 5,50 (d, J = 6 Hz, 1H : CH 1α) ; 5,72 (dd, J = 10,5 et 4 Hz, 1H : CH α d'une leucine) ; 6,22 (s, 1H : CH 3α) ; 7,18 (d, J = 8 Hz, 1H : CONH en 8) ; 7,38 (d, J = 9 Hz, 1H : CONH en 5) ; 7,70 (d, J = 7,5 Hz, 1H : CONH en 7) ; 7,95 (d, J = 9,5 Hz, 1H : CONH en 2).

Le disulfure de di-[2-(1-pipéridyl)-éthyle] peut être préparé selon la méthode décrite par R.C. Fuson dans J. Org. Chem., 11, 487 (1946).

### Exemple 4

La [(R)-2-(N-méthyl-N-i.propylamino)éthylthio-Sar]³ cyclosporine A est préparée selon la méthode suivante :

A 100 cm³ d'ammoniac maintenus à une température voisine de -33°C, on ajoute 100 mg de sodium métallique puis 100 mg de nitrate ferrique. Dès que la coloration bleue du mélange a disparu, 1,1 g de sodium métallique sont ajoutés en 15 minutes. Le mélange est agité à -33°C pendant 2 heures, une solution de 3,6 g de cyclosporine A dans 60 cm³ de tétrahydrofuranne est ajoutée goutte à goutte en approximativement 20 minutes puis une solution de 3,1 g de disulfure de di-[2-(N-méthyl-N-i.propylamino)éthyle] dans 15 cm³ de tétrahydrofuranne est ajoutée en 15 minutes. Le mélange réactionnel est agité à une température voisine de -33°C durant 1 heure, puis 3 g de chlorure d'ammonium solide sont ajoutés par portions. Sous agitation, on laisse s'évaporer l'ammoniac en faisant passer la température du mélange de -33 à 25°C en 12 heures. Le mélange est dilué par 100 cm³ d'éther diéthylique puis filtré. Le solide est rincé avec 100 cm³ au total d'éther diéthylique. Les phases organiques réunies sont concentrées sous pression réduite (2,7 kPa), à une température voisine de 40°C. Le solide obtenu (6,3 g) est purifié par chromatographie sur une colonne de silice (0,020-0,045 mm) (éluant : acétate d'éthyle-méthanol 4:1 en volumes) et en recueillant des fractions de 50 cm³. Les fractions contenant le produit attendu (fractions 32 à 48) sont réunies et concentrées sous pression réduite (2,7 kPa) à une température voisine de 40°C pour donner 0,640 g d'une laque incolore qui, traitée par 30 cm³ d'eau distillée donne après filtration et séchage à une température voisine de 40°C, 0,390 g de [(R)-2-(N-méthyl-N-i.propylamino)éthylthio-Sar]³ cyclosporine A sous la forme d'un solide blanc cassé fondant vers 70°C.

Spectre de R.M.N. ¹H (400 MHz, CDCl₃, δ en ppm) : 1,26 (d, J = 7,5 Hz, 3H : CH₃ 8β) ; 1,37 (d, J = 7,5 Hz, 3H : CH₃ 7β) ; 2,18 (s, 3H : NCH₃ du 2 N-méthyl N-isopropyl aminoéthylthio en 3α) ; de 2,55 à 2,75 (mt : les 4H correspondant au SCH₂CH₂N du 2 N-méthyl N-isopropyl aminoéthylthio en 3α) ; 2,71 - 2,72 - 3,12 - 3,14 - 3,27 - 3,45 et 3,51 (7 s, 3H chacun : les 7 NCH₃) ; 2,83 (mt, 1H : NCH du 2 N-méthyl N- isopropyl aminoéthylthio en 3α) ; 3,65 (d, J = 6 Hz, 1H : OH en 1β) ; 3,77 (mt, 1H : CH 1β) ; 4,54 (mt, 1H : CH 7α) ; 4,65 (t large, J = 9 Hz, 1H : CH 5α) ; 4,84 (mt, 1H : CH 8α) ; 4,97 (dd, J = 10,5 et 6 Hz, 1H : CH α d'une leucine) ; de 5,00 à 5,10 (mt, 2H : CH α d'une leucine et CH 2α) ; 5,13 (d, J = 11 Hz, 1H : CH 11α) ; 5,24 (dd, J = 11,5 et 4 Hz, 1H : CH α d'une leucine) ; 5,33 (mt, 2H : CH=CH) ; 5,50 (d, J = 6 Hz, 1H : CH 1α) ; 5,71 (dd, J = 10,5 et 4 Hz, 1H : CH α d'une leucine) ; 5,97 (s, 1H : CH 3α) ; 7,17 (d, J = 8 Hz, 1H : CONH en 8) ; 7,34 (d, J = 9 Hz, 1H : CONH en 5) ; 7,66 (d, J = 8 Hz, 1H : CONH en 7) ; 7,95 (d, J = 10 Hz, 1H : CONH en 2).

Le disulfure de di[2-(N-méthyl-N-i.propylamino)éthyle peut être préparé de la manière suivante:

A une solution de 20 g de 2-(N-i.propyle-N-méthylamino)-éthanethiol dans 150 cm³ d'éther de diéthyle, on ajoute 60 cm³ d'une solution aqueuse 5N d'hydroxyde de sodium puis on fait passer un courant d'air durant 12 heures à une température voisine de 20°C. Le mélange est extrait-au moyen de 150 cm³ au total d'éther de diéthyle. Les phases organiques réunies sont lavées par 100 cm³ d'eau distillée, séchées sur sulfate de magnésium puis concentrées sous pression réduite (2,7 kPa) à une température voisine de 40°C, pour conduire à 11,1 g de disulfure de di-[2-(N-méthyl-N-i.propylamino)éthyle] sous la forme d'une huile incolore.

Le 2-(N-isopropyle-N-méthylamino)-éthanethiol est préparé selon la méthode suivante:

Une solution de 115 cm³ de N-i.propyl-N-méthylamine et de 44 cm³ d'épisulfure éthylène dans 400 cm³ d'éther diéthylique est chauffée à une température voisine du reflux durant 36 heures. Une distillation fractionnée sous pression réduite (2,5 kPa) du mélange réactionnel conduit à 43 g de 2-(N-i.propyl-N-méthylamino)-éthanethiol sous la forme d'une huile incolore bouillant vers 60°C sous 2,5 kPa.

### Exemple 5

La [(R)-2-(N-méthyl-N-t.butylamino)éthylthio-Sar]³ cyclosporine A est préparée selon la méthode suivante:

A 100 cm³ d'ammoniac maintenus à une température voisine de -33°C, on ajoute 100 mg de sodium métallique puis 100 mg de nitrate ferrique. Dès que la coloration bleue du mélange a disparu, 1,0 g de sodium métallique est ajouté en 30 minutes. Le mélange est agité à -33°C pendant 1 heure, une solution de 3,6 g de cyclosporine A dans 50 cm³ de tétrahydrofuranne est ajoutée goutte à goutte en approximativement 30 minutes puis une solution de 3,5 g de disulfure de di-[2-(N-méthyl-N-t.butylamino)éthylel dans 15 cm³ de tétrahydrofuranne est ajoutée en 15 minutes. Le mélange réactionnel est agité à une température voisine de -33°C durant 1 heure, puis 3 g de chlorure d'ammonium solide sont ajoutés par portions. Sous agitation, on laisse s'évaporer l'ammoniac en faisant passer la température du mélange de -33 à 25°C en 12 heures. Le mélange est dilué par 100 cm³ d'éther diéthylique puis filtré. Le solide est rincé avec 300 cm³ au total d'éther diéthylique. Les phases organiques réunies sont concentrées sous pression réduite (2,7 kPa), à une température voisine de 40°C. Le solide obtenu est trituré avec 250 cm³ de pentane puis filtré. Le solide résiduel (6 g) est purifié par chromatographie sur une colonne de silice (0,020-0,045 mm) (éluant : acétate d'éthyle-méthanol 4:1 en volumes) et en recueillant des fractions de 100 cm³. La fraction contenant le produit attendu (fraction 9) est concentrée sous pression réduite (2,7 kPa) à une température voisine de 40°C pour donner 0,500 g d'un solide qui est agité avec 30 cm³ de pentane et donne, après filtration et séchage à une température voisine de 40°C, 0,200 g de [(R)-2-(N-méthyl-N-t-butylamino)éthylthio-Sar]³ cyclosporine A sous la forme d'un solide blanc cassé fondant vers 130°C.

Spectre de R.M.N. ¹H (400 MHz, CDCl₃, δ en ppm) : 1,05 (s : les 9H correspondant au C(CH₃)₃) ; 1,26 (d, J = 7,5 Hz, 3H : CH₃ 8β) ; 1,36 (d, J = 7,5 Hz, 3H : CH₃ 7β) ; 1,63 (d, J = 5 Hz, 3H : CH₃ 1η) ; 2,19 (s, 3H : NCH₃ du 2 N-terbutyl N-méthyl aminoéthylthio en 3α) ; de 2,55 à 2,80 (mt : les 4H correspondant au SCH₂CH₂N du 2 N-terbutyl N-méthyl aminoéthylthio en 3α) ; 2,70 - 2,72 - 3,12 - 3,13 - 3,26 - 3,45 et 3,51 (7 s, 3H chacun : les 7 NCH₃) ; 3,61 (d, J = 6,5 Hz, 1H : OH en 1β) ; 3,76 (mt, 1H : CH 1β) ; 4,54 (mt, 1H : CH 7α) ; 4,65 (t large, J = 9 Hz, 1H : CH 5α) ; 4,84 (mt, 1H : CH 8α) ; 4,97 (dd, J = 10 et 6 Hz, 1H : CH α d'une leucine) ; de 5,00 à 5,10 (mt, 2H : CH α d'une leucine et CH 2α) ; 5,13 (d, J = 11 Hz, 1H : CH 11α) ; 5,24 (dd, J = 10,5 et 4 Hz, 1H : CH α d'une leucine) ; 5,34 (mt, 2H : CH=CH) ; 5,49 (d, J = 6 Hz, 1H : CH 1α) ; 5,71 (dd, J = 10,5 et 4 Hz, 1H : CH α d'une leucine) ; 5,90 (s, 1H : CH 3α) ; 7,16 (d, J = 8 Hz, 1H : CONH en 8) ; 7,33 (d, J = 9 Hz, 1H : CONH en 5) ; 7,66 (d, J = 8 Hz, 1H : CONH en 7) ; 7,96 (d, J = 10 Hz, 1H : CONH en 2).

Le disulfure de di-[2-(N-méthyl-N-t.butylamino)éthyle] peut être préparé de la manière suivante:

A une solution de 28,7 g de 2-(N-t.butyl-N-methylamino)-éthanethiol dans 190 cm³ de méthanol, on ajoute 0,1 cm³ d'une solution aqueuse 1N d'hydroxyde de sodium puis on fait passer un courant d'air durant 60 heures à une température voisine de 20°C. Le méthanol est éliminé sous pression réduite (2,7 kPa). L'huile résiduelle est mise en solution dans 400 cm³ d'éther de diéthyle. La phase organique est séchée sur sulfate de magnésium, filtrée puis concentrée sous pression réduite (2,7 kPa) à une température voisine de 40°C, pour conduire à 26,6 g de disulfure de di-[2-(N-méthyl-N-t.butylamino) éthyle] sous la forme d'une huile jaune.

Le 2-(N-t.butyl-N-méthylamino)-éthanethiol peut être préparé selon la méthode suivante:

Une solution de 125 cm³ de N-t.butyl-N-méthylamine et de 50 g d'épisulfure éthylène dans 750 cm³ de t.butyl méthyl éther est agitée durant 48 heures à une température voisine du reflux. Le mélange est concentré sous pression réduite (10 kPa) à une température voisine de 35°C. Une distillation fractionnée sous pression réduite (5,8 kPa) du mélange réactionnel conduit à 28,7 g de 2-(N-t.butyl-N-méthylamino)-éthanethiol sous la forme d'une huile incolore bouillant entre 84 et 86°C sous 5,8 kPa.

### Exemple 6

La [(R)-2-(1-imidazolyl)-éthylthio-Sar]³ cyclosporine A est préparée selon la méthode suivante:

A 80 cm³ d'ammoniac maintenus à une température voisine de -33°C, on ajoute 100 mg de sodium métallique puis 100 mg de nitrate ferrique.
Dès que la coloration bleue du mélange a disparu, 0,82 g de sodium métallique sont ajoutés en 15 minutes. Le mélange est agité à -33°C pendant 15 minutes, ensuite une solution de 2,4 g de cyclosporine A dans 10 cm³ de tétrahydrofuranne est ajoutée goutte à goutte en approximativement 15 minutes puis 5 g de disulfure de di-[2-(1-imidazolyl)éthyle] solide sont ajoutés par portions en 15 minutes.
Le mélange réactionnel est agité à une température voisine de -33°C durant 3 heures, puis 3,4 g de chlorure d'ammonium solide sont ajoutés par portions. Sous agitation, on laisse s'évaporer l'ammoniac en faisant passer la température du mélange de -33 à 25°C en 12 heures. Le mélange est dilué par 100 cm³ d'eau distillée. La phase organique est décantée, la phase aqueuse est lavée par 3 fois 50 cm³ d'acétate d'éthyle. Les phases organiques réunies sont séchées sur sulfate de magnésium, filtrées puis concentrées sous pression réduite (2,7 kPa), à une température voisine de 40°C. La meringue beige ainsi obtenue (2,25 g) est purifiée par chromatographie sur une colonne de silice (0,020-0,045 mm) (éluant : acétate d'éthyle-méthanol 19:1 en volumes) et en recueillant des fractions de 20 cm³.
Les fractions contenant le produit attendu sont concentrées sous pression réduite (2,7 kPa) à une température voisine de 40°C pour donner 0,520 g d'un solide. Ce solide, trituré avec 50 cm³ de pentane donne après filtration et séchage à une température voisine de 40°C, 0,420 g d'un solide qui est purifié par chromatographie sur une seconde colonne de silice (0,020-0,045 mm) (éluant : acétate d'éthyle-méthanol 4:1 en volumes). Les fractions contenant le produit attendu sont concentrées sous pression réduite (2,7 kPa) à une température voisine de 40°C. Le solide résiduel est agité avec 10 cm³ de pentane pour donner, après filtration est séchage à une température voisine de 40°C, 0,245 g de [(R)-2-(1-imidazolyl)-éthylthio-Sar]³ cyclosporine A sous la forme d'un solide jaune fondant vers 208°C.

Spectre de R.M.N. ¹H (400 MHz, CDCl₃, à une température de 333 K, δ en ppm) : 1,26 (d, J = 7,5 Hz, 3H : CH₃ 8β) ; 1,36 (d, J = 7,5 Hz, 3H : CH₃ 7β) ; 1,63 (d, J = 5 Hz, 3H : CH₃ 1η) ; 2,72 - 3,07 - 3,15 - 3,25 - 3,40 et 3,50 (6 s, respectivement 6H - 3H - 3H - 3H - 3H - 3H : les 7 NCH₃) ; de 3,80 à 3,95 (mt, 2H : CH 1β et OH en 1β) ; 4,16 (mt, 2H : NCH₂) ; 4,51 (mt, 1H : CH 7α) ; 4,71 (t large, J = 9 Hz, 1H : CH 5α) ; 4,86 (mt, 1H : CH 8α) ; de 4,95 à 5,10 (mt, 3H : CH α de deux leucines et CH 2α) ; 5,17 (d, J = 11 Hz, 1H : CH 11α) ; 5,22 (dd, J = 11,5 et 4 Hz, 1H : CH α d'une leucine) ; 5,35 (mt, 2H : CH=CH) ; 5,42 (d, J = 6 Hz, 1H : CH 1α) ; 5,72 (dd, J = 10,5 et 4 Hz, 1H : CH α d'une leucine) ; 5,82 (s, 1H : CH 3α) ; 6,93 - 7,10 et 7,54 (3 s larges, 1H chacun : H aromatiques de l'imidazole) ; 7,12 (d, J = 8 Hz, 1H : CONH en 8) ; 7,19 (d, J = 9 Hz, 1H : CONH en 5) ; 7,53 (mt, 1H : CONH en 7) ; 7,87 (d, J = 10 Hz, 1H : CONH en 2).

Le disulfure de di-[2-(1-imidazolyl)éthyle] peut être préparé de la manière suivante:

A une solution de 15 g de 2-(1-imidazolyl)-éthanethiol dans 200 cm³ de dichlorométhane refroidie à 0°C, on ajoute goutte à goutte en 10 minutes, 32,3 cm³ de triéthylamine puis une solution de 14,59 g d'iode dans 68 cm³ d'éther de diéthyle. Le mélange est agité durant 30 minutes, à une température voisine de 20°C puis concentré sous pression réduite (2,7 kPa) à une température voisine de 40°C. Le résidu pâteux est trituré en présence de 50 cm³ d'isopropanol. Le solide formé est filtré et rincé avec 25 cm³ au total d'isopropanol pour donner un premier jet de disulfure de di-[2-(1-imidazolyl)éthyle]. Les phases organiques réunies sont concentrées sous pression réduite (2,7 kPa) à une température voisine de 40°C.

Le résidu pâteux est trituré en présence de 50 cm³ d'acétate d'éthyle. Le solide formé est filtré pour donner un second jet de disulfure de di-[2-(1-imidazolyl)éthyle]. Les deux jets de disulfure de di-[2-(1-imidazolyl)éthyle] sont réunis et séchés sous vide (10 kPa) à une température voisine de 20°C pour conduire à 14,2 g de disulfure de di-[2-(1-imidazolyl)éthyle].

Le 2-(1-imidazolyl)-éthanethiol peut être préparé de la manière suivante:

Une solution de 28,34 g de chlorhydrate de 2-(1-imidazolyl)éthyl-isothiourée et de 18,56 g d'hydroxyde de sodium dans 300 cm³ d'eau distillée est chauffée à reflux durant 150 minutes. Après avoir été raméné à une température voisine de 20°C, le mélange est acidifié par ajout d'acide chlorhydrique concentré (20 cm³) puis amené à pH = 7 par ajout d'une solution saturée de bicarbonate de sodium. Le mélange est extrait par 600 cm³ au total d'acétate d'éthyle. Les phases organiques réunies sont lavées par 100 cm³ au total d'eau distillée, filtrées, séchées sur sulfate de magnésium puis concentrées sous pression réduite (2,7 kPa) à une température voisine de 40°C pour conduire à 15,0 g de 2-(1-imidazolyl)-éthanethiol sous la forme d'une huile jaune.

Le chlorhydrate de 2-(1-imidazolyl)éthylisothiourée peut être préparé de la manière suivante:

A une solution de 30 g de 2-hydroxy-(1-imidazolyl)-éthane dans 300 cm³ de dichlorométhane on ajoute goutte à goutte en 30 minutes 44,2 cm³ de chlorure de thionyle puis on porte le mélange à une température voisine du reflux durant 16 heures. Le mélange est concentré sous pression réduite (2,7 kPa) à une température voisine de 40°C. Le residu pâteux est traité par 100 cm³ de dichlorométhane puis concentré sous pression réduite (2,7 kPa) à une température voisine de 40°C. Cette étape est répétée deux fois. Une suspension de 34,9 g de chlorhydrate de 2-chloro-(1-imidazolyl)-éthane brut ainsi obtenu et 15,93 g de thiourée dans 125 cm³ de diméthylformamide est chauffée à une température voisine de 110°C durant 90 minutes.

Le mélange est refroidi à une température voisine de 20°C. Le solide jaune formé est ensuite filtré, rincé avec 100 cm³ au total d'éther de diéthyle et séché sous vide (10 kPa) à une température voisine de 40°C pour conduire à 28,34 g de chlorhydrate de 2-(1-imidazolyl)éthylisothiourée sous la formé d'un solide fondant à une température voisine de 206°C.

Le 2-hydroxy-(1-imidazolyl)-éthane peut être préparé de la manière suivante:

A une suspension de 30 g d'hydrure de sodium (à 50% dans l'huile minérale) dans 250 cm³ de diméthylformamide on ajoute en 30 minutes une solution de 68 g d'imidazole de 250 cm³ de diméthylformamide. Le mélange est agité 90 minutes à une température voisine de 20°C puis une solution de 50,5 g de 2-chloroéthanol dans 50 cm³ de diméthylformamide est ajoutée en une heure. Le mélange est agité 12 heures à une température voisine de 20°C puis filtré. Le filtrat est traité par 100 cm³ d'eau distillée puis concentré sous pression réduite (2,7 kPa) à une température voisine de 55°C. Le résidu pâteux est repris par 150 cm³ d'éther de pétrole, la phase liquide est décantée et le résidu est trituré durant une heure avec 100 cm³ d'isopropanol. Le précipité formé est filtré, le filtrat est concentrée sous pression réduite (2,7 kPa) à une température voisine de 40°C. L'huile résiduelle (113,1 g) est distillée sous pression réduite (5 kPa) pour conduire à 105,7 g de 2-hydroxy-(1-imidazolyl)-éthane sous la forme d'une huile jaune distillant à une température de 180-183°C sous 5 kPa.

### Exemple 7

En opérant de manière analogue aux méthodes d'écrites dans les exemples 1 à 3, on prépare les produits suivants :
[(R)-2-aminoéthylthio-Sar]³-cyclosporine A ;
[(R)-2-(N-méthylamino)éthylthio-Sar]³-cyclosporine A ;
[(R)-2-(N-éthylamino)éthylthio-Sar]³-cyclosporine A ;
[(R)-2-(N-i.propylamino)éthylthio-Sar]³-cyclosporine A ;
[(R)-2-(N-t.butylamino)éthylthio-Sar]³-cyclosporine A ;
[(R)-2-(N-phénylamino)éthylthio-Sar]³-cyclosporine A ;
[(R)-2-(N-benzylamino)éthylthio-Sar]³-cyclosporine A ;
[(R)-2-(N-méthyl-N-éthylamino)éthylthio-Sar]³-cyclosporine A ;
[(R)-2-(N-méthyl-N-allylamino)éthylthio-Sar]³-cyclosporine A ;
[(R)-2-(N-méthyl-N-phénylamino)éthylthio-Sar]³-cyclosporine A ;
[(R)-2-(N-méthyl-N-benzylamino)éthylthio-Sar]³-cyclosporine A ;
[(R)-2-(N,N-di-i.propylamino)éthylthio-Sar]³-cyclosporine A ;
[(R)-2-(N,N-diallylamino)éthylthio-Sar]³-cyclosporine A ;
[(R)-3-aminopropylthio-Sar]³-cyclosporine A ;
[(R)-3-(N-méthylamino)propylthio-Sar]³-cyclosporine A ;
[(R)-3-(N-éthylamino)propylthio-Sar]³-cyclosporine A ;
[(R)-3-(N-i.propylamino)propylthio-Sar]³-cyclosporine A ;
[(R)-3-(N-t.butylamino)propylthio-Sar]³-cyclosporine A ;
[(R)-3-(N-phénylamino)propylthio-Sar]³-cyclosporine A ;
[(R)-3-(N-benzylamino)propylthio-Sar]³-cyclosporine A ;
[(R)-3-(N-méthyl-N-éthylamino)propylthio-Sar]³-cyclosporine A ;
[(R)-3-(N-méthyl-N-i.propyllamino)propylthio-Sar]³-cyclosporine A ;
[(R)-3-(N-méthyl-N-t.butylamino)propylthio-Sar]³-cyclosporine A ;
[(R)-3-(N-méthyl-N-allylamino)propylthio-Sar]³-cyclosporine A ;
[(R)-3-(N-méthyl-N-phénylamino)propylthio-Sar]³-cyclosporine A ;
[(R)-3-(N-méthyl-N-benzylamino)propylthio-Sar]³-cyclosporine A ;
[(R)-2-(N,N-diméthylamino)éthylthio-Sar]³-cyclosporine A ;
[(R)-3-(N,N-diéthylamino)propylthio-Sar]³-cyclosporine A ;
[(R)-3-(N,N-di-i.propylamino)propylthio-Sar]³-cyclosporine A ;
[(R)-3-(N,N-diallylamino)propylthio-Sar]³-cyclosporine A ;
[(R)-3-(1-pipéridyl)propylthio-Sar]³-cyclosporine A ;
[(R)-4-aminobutylthio-Sar]³-cyclosporine A ;
[(R)-4-(N-méthylamino)butylthio-Sar]³-cyclosporine A ;
[(R)-4-(N-éthylamino)butylthio-Sar]³-cyclosporine A ;
[(R)-4-(N-i.propylamino)butylthio-Sar]³-cyclosporine A ;
[(R)-4-(N-t.butylamino)butylthio-Sar]³-cyclosporine A ;
[(R)-4-(N-phénylamino)butylthio-Sar]³-cyclosporine A ;
[(R)-4-(N-benzylamino)butylthio-Sar]³-cyclosporine A ;
[(R)-4-(N-méthyl-N-éthylamino)butylthio-Sar]³-cyclosporine A ;
[(R)-4-(N-méthyl-N-i.propylamino)butylthio-Sar]³-cyclosporine A ;
[(R)-4-(N-méthyl-N-t.butylamino)butylthio-Sar]³-cyclosporine A ;
[(R)-4-(N-méthyl-N-allylamino)butylthio-Sar]³-cyclosporine A ;
[(R)-4-(N-méthyl-N-phénylamino)butylthio-Sar]³-cyclosporine A ;
[(R)-4-(N-méthyl-N-benzylamino)butylthio-Sar]³-cyclosporine A ;
[(R)-4-(N,N-diméthylamino)butylthio-Sar]³-cyclosporine A ;
[(R)-4-(N,N-diéthylamino)butylthio-Sar]³-cyclosporine A ;
[(R)-4-(N,N-di-i.propylamino)butylthio-Sar]³-cyclosporine A ;
[(R)-4-(N,N-diallylamino)butylthio-Sar]³-cyclosporine A ;
[(R)-4-(1-pipéridyl)butylthio-Sar]³-cyclosporine A ;
[(R)-2-amino-2-méthylpropylthio-Sar]³-cyclosporine A ;
[(R)-2-(N,N-diméthylamino)-2-méthylpropylthio-Sar]³-cyclosporine A ;
[(R)-2-(N,N-diéthylamino)-2-méthylpropylthio-Sar]³-cyclosporine A ;
[(R)-2-(1-pipéridyl)-2-méthylpropylthio-Sar]³-cyclosporine A ;
[(R)-3-amino-3-méthylbutylthio-Sar]³-cyclosporine A ;
[(R)-3-(N,N-diméthylamino)-3-méthylbutylthio-Sar]³-cyclosporine A ;
[(R)-3-(N,N-diéthylamino)-3-méthylbutylthio-Sar]³-cyclosporine A ;
[(R)-3-(1-pipéridyl)-3-méthylbutylthio-Sar]³-cyclosporine A ;
[(R)-2-(1-morpholino)éthylthio-Sar]³-cyclosporine A ;
[(R)-2-(1-azétidino)éthylthio-Sar]³-cyclosporine A ;
{(R)-2-[1-(4-méthylpipérazino)}éthylthio-Sar}³-cyclosporine A ;
{(R)-2-[1-(4-phénylpipérazino)]éthylthio-Sar}³-cyclosporine A ;
{(R)-2-[1-(4-benzylpipérazino)]éthylthio-Sar}³-cyclosporine A ;
{(R)-2-[1-(4-méthyl-1,2,3,6-tétrahydropyridyl)]éthylthio-Sar}³-cyclosporine A ;
{(R)-2-[1-(4-phényl-1,2,3,6-tétrahydropyridyl)]éthylthio-Sar}³-cyclosporine A ;
[(R)-3-(1-morpholino)propylthio-Sar]³-cyclosporine A ;
[(R)-3-(1-azétidino)propylthio-Sar]³-cyclosporine A ;
{(R)-3-[1-(4-méthylpipérazino)]propylthio-Sar}³-cyclosporine A ;
{(R)-3-[1-(4-phénylpipérazino)]propylthio-Sar}³-cyclosporine A ;
{(R)-3-[1-(4-benzylpipérazino)]propylthio-Sar}³-cyclosporine A ;
{(R)-3-[1-(4-méthyl-1,2,3,6-tétrahydropyridyl)]propylthio-Sar}³-cyclosporine A ;
{(R)-3-[1-(4-méthyl-1,2,3,6 tétrahydropyridyl)]propylthio-Sar}³-cyclosporine A.

La présente invention concerne également les compositions pharmaceutiques contenant au moins un produit de formule générale (I) le cas échéant sous forme de sel, à l'état pur ou sous forme d'une association avec un ou plusieurs diluants ou adjuvants compatibles et pharmaceutiquement acceptables, ou avec un autre agent antirétrovirus, éventuellement destiné au traitement du SIDA, un agent antiviral, immunomodulateur ou antimicrobien.

La composition selon l'invention est capable de maintenir en vie les cellules infectées par un rétrovirus comme par exemple le VIH et donc de réduire la progression vers le SIDA ou de diminuer sa gravité chez les sujets déjà infectés en réduisant la mortalité des cellules infectées. Les compositions peuvent être utilisées par voie orale, parentérale, rectale ou en aérosols.

Les compositions pharmaceutiques peuvent être utilisées à titre curatif ou à titre préventif chez des sujets présentant une immunodéficience et/ou infectés par un rétrovirus. Bien entendu, la constitution de ces compositions sera adaptée au cas particulier du tractus digestif des immunodéprimés.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon.

Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle.

Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre mélange stérile injectable.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogéne, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, le médecin déterminera la posologie qu'il estime la plus appropriée en fonction d'un traitement préventif ou curatif, en fonction de l'âge, du poids, du degré de l'infection et des autres facteurs propres au sujet à traiter. Généralement, les doses sont comprises entre 5 et 30 mg/kg par voie orale pour un adulte.

Il a de plus été montré que les dérivés de cyclosporine de formule générale (I) manifestent un effet de synergie ou au moins d'addition lorsqu'ils sont associés à d'autres agents anti-viraux actifs sur les rétrovirus. La présente invention concerne également les associations synergisantes qui contiennent au moins un dérivé de la cyclosporine de formule générale (I) et/ou le cas échéant leurs sels, et un principe actif connu pour son activité sur les rétrovirus.

Les agents connus pour leur activité sur les rétrovirus qui peuvent être associés, sont choisis parmi des agents compatibles et inertes vis à vis du dérivé de cyclosporine de formule générale (I), aussi bien dans la catégorie des traitements pharmacologiques que dans la catégorie des traitements alternatifs tels que la thérapie génique et cellulaire ou antisens. A titre non limitatif ces agents constituant les differentes classes thérapeutiques sont choisis par exemple parmi des inhibiteurs nucléosidiques (NRTI) et non nucléosidiques (NNRTI) de la reverse transcriptase [zidovudine (AZT), didanosine (DDI), didéoxycytidine (DDC), d4T, ribavirine, 3TC, névirapine ...], parmi des inhibiteurs de la protéase [comme par exemple le Saquinavir, le Ritonavir, l'Indinavir et le Nelfinavir], des inhibiteurs de l'intégrase [comme le AR177], parmi les inhibiteurs de gene thérapie ciblant les protéines régulatrices de la réplication des VIHs tels que les inhibiteurs de la protéine rev [comme par exemple le Rev M10], ou les inhibiteurs de la nucléocapside [comme par exemple les DIBAs], parmi les inhibiteurs ciblant les transcripts RNA messager specifiques de tous les VIHs comme par exemple les anti-sens [comme GEM92, GPI-2A...], parmi les inhibiteurs de la famille des modulateurs de dNTP cellulaire [comme hydroxyurée], parmi les inhibiteurs de cytokines [comme TNF], parmi les inhibiteurs d'entrée des VIHs [comme T20, SPC-3...], ainsi que parmi les agents constituants les classes therapeutiques utilisées dans les approches vaccinales tant par biotechnologie [comme HIVAC-le, ALVAC...] que par composés agissant sur la réponse immune [comme RG-8394].

Notamment le dérivé de cyclosporine de l'exemple 2 manifeste un effet particulièrement intéressant lorsqu'il est associé avec l'AZT, le ddI, le Sasquinavir, et la ribavirine.

Les compositions pharmaceutiques comprenant de telles associations, éventuellement en présence d'excipients pharmaceutiquement acceptables, entrent également dans le cadre de la présente invention.

L'exemple suivant illustre une composition selon l'invention.

### Exemple

On prépare une formulation administrable par voie orale et ayant la composition suivante :

| | |
|---|---|
| [(R)-2-(N,N-diméthylamino)éthylthio-Sar]³-cyclosporine A | 250 mg |
| Stéarate de magnésium | 3 mg |
| Acidsol | 15 mg |
| Silice colloïdale | 2 mg |
| Lactose | 130 mg |

## Revendications

1. Un dérivé de la cyclosporine **caractérisé en ce qu'**il répond à la formule générale : 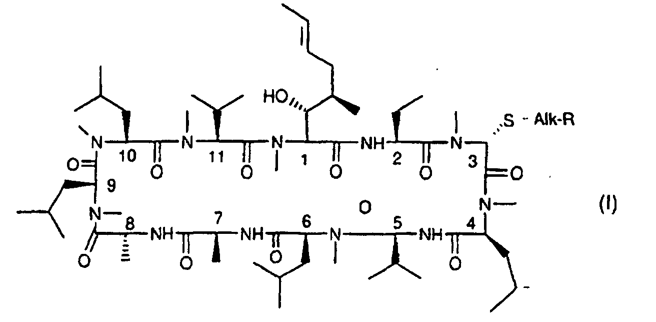 dans laquelle :
- Alk représente un radical alcoylène contenant 2 à 6 atomes de carbone en chaîne droite ou ramifiée ou cycloalcoylène contenant 3 à 6 atomes de carbone et
- R représente
• soit un radical carboxy ou alcoyloxycarbonyle,
• soit un radical -NR₁R₂ pour lequel R₁ et R₂ identiques ou différents représentent des atomes d'hydrogène, ou des radicaux alcoyle, alcènyle (2 à 4C), cycloalcoyle (3 à 6C), phényle éventuellement substitué (par un atome d'halogène, alcoyloxy, alcoyloxycarbonyle, amino, alcoylamino ou dialcoylamino), ou représentent des radicaux benzyle ou hétérocyclyle saturé ou insaturé contenant 5 ou 6 chaînons et 1 à 3 hétéroatomes, ou pour lequel R₁ et R₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alcoyle, phényle ou benzyle,
. soit un radical de formule générale : 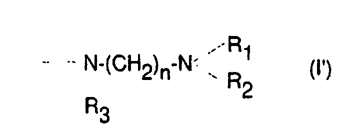 pour lequel R₁ et R₂ sont définis comme ci-dessus, R₃ représente un atome d'hydrogène ou un radical alcoyle et n est un nombre entier de 2 à 4,
les portions ou radicaux alcoyle définis ci-dessus étant droits ou ramifiés et contenant 1 à 4 atomes de carbone, ainsi que ses sels pharmaceutiquement acceptables lorsqu'ils existent.

2. Un dérivé de la cyclosporine selon la revendication 1,
**caractérisé en ce que** :
- Alk représente un radical alcoylène contenant 2 à 6 atomes de carbone en chaîne droite ou ramifiée et
- R représente un radical -NR₁R₂ pour lequel R₁ et R₂ identiques ou différents représentent des atomes d'hydrogène, ou des radicaux alcoyle, alcènyle (2 à 4C), phényle éventuellement substitué (par un atome d'halogène, alcoyloxy, alcoyloxycarbonyle, amino, alcoylamino ou dialcoylamino), ou représentent des radicaux benzyle ou pour lequel R₁ et R₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alcoyle,
les portions ou radicaux alcoyle définis ci-dessus étant droits ou ramifiés et contenant 1 à 4 atomes de carbone, ainsi que ses sels pharmaceutiquement acceptables lorsqu'ils existent.

3. Un dérivé de la cyclosporine selon la revendication 1,
**caractérisé en ce que** :
- Alk représente un radical alcoylène contenant 2 à 5 atomes de carbone en chaine droite ou ramifiée et
- R représente un radical -NR₁R₂ pour lequel R₁ et R₂ identiques ou différents représentent des atomes d'hydrogène, ou des radicaux alcoyle, allyle, phényle ou benzyle ou pour lequel R₁ et R₂ forment avec l'atome d'azote auquel ils sont rattachés un hétérocycle choisi parmi azétidinyle, pipéridyle, pipérazinyle, N-méthyl pipérazinyle, N-phényl pipérazinyle, N-benzyl pipérazinyle, imidazolyle, morpholino, tétrahydropyridyle, méthyl tétrahydropyridyle, phényl tétrahydropyridyle.
les portions ou radicaux alcoyle définis ci-dessus étant droits ou ramifiés et contenant 1 à 4 atomes de carbone, ainsi que ses sels pharmaceutiquement acceptables lorsqu'ils existent.

4. Un dérivé de la cyclosporine selon la revendication 1, **caractérisé en ce qu'**il s'agit de la [(R)-2-(N,N-diéthylamino)éthylthio-Sar]³-cyclosporine A, ainsi que ses sels pharmaceutiquement acceptables.

5. Un dérivé de la cyclosporine selon la revendication 1, **caractérisé en ce qu'**il s'agit de la [(R)-2-(N,N-diméthylamino)éthylthio-Sar]³-cyclosporine A, ainsi que ses sels pharmaceutiquement acceptables.

6. Un dérivé de la cyclosporine selon la revendication 1, **caractérisé en ce qu'**il s'agit de la [(R)-2-(1-pipéridyl)-éthylthio-Sar]³ cyclosporine A, ainsi que ses sels pharmaceutiquement acceptables.

7. Un dérivé de la cyclosporine selon la revendication 1, **caractérisé en ce qu'**il s'agit de la [(R)-2-(N-méthyl-N-i.propyl-amino)éthylthio-Sar]³ cyclosporine A, ainsi que ses sels pharmaceutiquement acceptables.

8. Un dérivé de la cyclosporine selon la revendication 1, **caractérisé en ce qu'**il s'agit de la [(R)-2-(N-méthyl-N-t.butylamino)éthylthio-Sar]³ cyclosporine A, ainsi que ses sels pharmaceutiquement acceptables.

9. Procédé de préparation d'un dérivé de cyclosporine selon la revendication 1, **caractérisé en ce que** l'on fait agir un disulfure de formule générale :
R-Alk-S-S-Alk-R
dans laquelle R et Alk sont définis comme ci-dessus, et dont le cas échéant les fonctions pouvant interférer avec la réaction ont été préalablement protégées, sur une forme activée de la cyclosporine A, puis élimine le cas échéant les radicaux protecteurs et/ou transforme éventuellement le produit obtenu en un sel lorsqu'ils existent.

10. Composition pharmaceutique **caractérisée en ce qu'**elle contient au moins un produit selon la revendication 1, à l'état pur ou en association avec tout diluant ou adjuvant compatible et pharmaceutiquement acceptable et/ou en association avec un autre principe actif antiviral, immunomodulateur ou antimicrobien.

11. Associations synergisantes **caractérisées en ce qu'**elles comprennent au moins un dérivé de cyclosporine selon la revendication 1 et au moins un autre agent connu pour son activité anti-rétrovirus.

## Patentansprüche

1. Ein Derivat des Cyclosporins, **dadurch gekennzeichnet, dass** es der allgemeinen Formel: 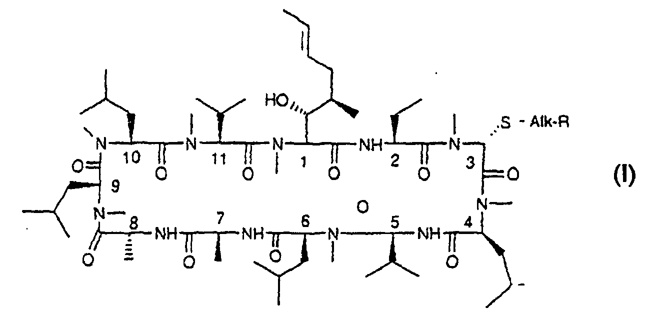 entspricht, worin:
- Alk einen Alkylenrest mit 2 bis 6 Kohlenstoffatomen in gerader oder verzweigter Kette oder Cycloalkylenrest mit 3 bis 6 Kohlenstoffatomen darstellt und
- R darstellt
• entweder einen Carboxy- oder Alkyloxycarbonylrest,
• oder einen Rest -NR₁R₂, für den R₁ und R₂, die gleich oder verschieden sind, Wasserstorfatome oder Reste Alkyl, Alkenyl (2 bis 4 C), Cycloalkyl (3 bis 6 C), Phenyl, gegebenenfalls substituiert (mit einem Halogenatom, Alkyloxy, Alkyloxycarbonyl, Amino, Alkylamino oder Dialkylamino), darstellen oder Reste Benzyl oder Heterocyclyl, gesättigt oder ungesättigt, mit 5 oder 6 Ringgliedern und 1 bis 3 Heteroatomen darstellen oder für den R₁ und R₂ mit dem Stickstoffatom, an das sie gebunden sind, einen Heterocyclus mit 4 bis 6 Ringgliedern, gesättigt oder ungesättigt, der ein weiteres Heteroatom enthalten kann, ausgewählt unter Stickstoff, Sauerstoff oder Schwefel, und gegebenenfalls substituiert mit Alkyl, Phenyl oder Benzyl, bilden;
• oder einen Rest der allgemeinen Formel: 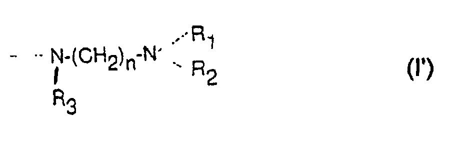 für den R₁ und R₂ wie oben definiert sind, R₃ ein Wasserstoffatom oder einen Alkylrest darstellt und n eine ganze Zahl von 2 bis 4 ist,
wobei die oben definierten Alkylteile oder -reste gerade oder verzweigt sind und 1 bis 4 Kohlenstoffatome enthalten, sowie seine pharmazeutisch annehmbaren Salze, wenn es welche gibt.

2. Ein Derivat des Cyclosporins gemäß Anspruch 1, **dadurch gekennzeichnet, dass**:
- Alk einen Alkylenrest mit 2 bis 6 Kohlenstoffatomen in gerader oder verzweigter Kette darstellt und
- R einen Rest -NR₁R₂ darstellt, für den R₁ und R₂, die gleich oder verschieden sind, Wasserstoffatome oder Reste Alkyl, Alkenyl (2 bis 4 C), Phenyl, gegebenenfalls substituiert (mit einem Halogenatom, Alkyloxy, Alkyloxycarbonyl, Amino, Alkylamino oder Dialkylamino), darstellen oder Benzylreste darstellen oder für den R₁ und R₂ mit dem Stickstoffatom, an das sie gebunden sind, einen Heterocyclus mit 4 bis 6 Ringgliedern, gesättigt oder ungesättigt, der ein weiteres Heteroatom enthalten kann, ausgewählt unter Stickstoff, Sauerstoff oder Schwefel, und gegebenenfalls substituiert mit Alkyl, bilden,
wobei die oben definierten Alkylteile oder -reste gerade oder verzweigt sind und 1 bis 4 Kohlenstoffatome enthalten, sowie seine pharmazeutisch annehmbaren Salze, wenn es welche gibt.

3. Ein Derivat des Cyclosporins gemäß Anspruch 1, **dadurch gekennzeichnet, dass**:
- Alk einen Alkylenrest mit 2 bis 5 Kohlenstoffatomen in gerader oder verzweigter Kette darstellt und
- R einen Rest -NR₁R₂ darstellt, für den R₁ und R₂, die gleich oder verschieden sind, Wasserstoffatome oder Reste Alkyl, Allyl, Phenyl oder Benzyl darstellen oder für den R₁ und R₂ mit dem Stickstoffatom, an das sie gebunden sind, einen Heterocyclus bilden, der ausgewählt ist unter Azetidinyl, Piperidyl, Piperazinyl, N-Methylpiperazinyl, N-Phenylpiperazinyl, N-Benzylpiperazinyl, Imidazolyl, Morpholino, Tetrahydropyridyl, Methyltetrahydropyridyl, Phenyltetrahydropyridyl,
wobei die oben definierten Alkylteile oder -reste gerade oder verzweigt sind und 1 bis 4 Kohlenstoffatome enthalten, sowie seine pharmazeutisch annehmbaren Salze, wenn es welche gibt.

4. Ein Derivat des Cyclosporins gemäß Anspruch 1, **dadurch gekennzeichnet, dass** es sich handelt um [(R)-2-(N,N-Diethylamino)ethylthio-Sar]³-cyclosporin A, sowie seine pharmazeutisch annehmbaren Salze.

5. Ein Derivat des Cyclosporins gemäß Anspruch 1, **dadurch gekennzeichnet, dass** es sich handelt um [(R)-2-(N,N-Dimethylamino)ethylthio-Sar]³-cyclosporin A, sowie seine pharmazeutisch annehmbaren Salze.

6. Ein Derivat des Cyclosporins gemäß Anspruch 1, **dadurch gekennzeichnet, dass** es sich handelt um [(R)-2-(1-Piperidyl)ethylthio-Sar]³-cyclosporin A, sowie seine pharmazeutisch annehmbaren Salze.

7. Ein Derivat des Cyclosporins gemäß Anspruch 1, **dadurch gekennzeichnet, dass** es sich handelt um [(R)-2-(N-Methyl-N-i-propylamino)ethylthio-Sar]³-cyclosporin A, sowie seine pharmazeutisch annehmbaren Salze.

8. Ein Derivat des Cyclosporins gemäß Anspruch 1, **dadurch gekennzeichnet, dass** es sich handelt um [(R)-2-(N-Methyl-N-t-butylamino)ethylthio-Sar]³-cyclosporin A, sowie seine pharmazeutisch annehmbaren Salze.

9. Verfahren zur Herstellung eines Cyclosporinderivats gemäß Anspruch 1,
**dadurch gekennzeichnet, dass** man ein Disulfid der allgemeinen Formel:
R-Alk-S-S-Alk-R
worin R und Alk wie oben definiert sind und bei dem gegebenenfalls die Funktionen, die die Reaktion beeinflussen können, zuvor geschützt wurden, auf eine aktivierte Form des Cyclosporins A einwirken lässt, dann gegebenenfalls die Schutzgruppen entfernt und/oder gegebenenfalls das erhaltene Produkt in ein Salz umwandelt, wenn es welche gibt.

10. Pharmazeutische Zusammensetzung, **dadurch gekennzeichnet, dass** sie wenigstens ein Produkt gemäß Anspruch 1 in reinem Zustand oder in Verbindung mit jedem kompatiblen und pharmazeutisch annehmbaren Verdünnungsmittel oder Hilfsstoff und/oder in Verbindung mit einem weiteren antiviralen, immunomodulatorischen oder antimikrobiellen Wirkstoff enthält.

11. Synergistische Kombinationen, **dadurch gekennzeichnet, dass** sie wenigstens ein Cyclosporinderivat gemäß Anspruch 1 und wenigstens ein weiteres Mittel, das für seine Anti-Retrovirus-Wirkung bekannt ist, umfassen.

## Claims

1. Cyclosporin derivative, **characterized in that** it corresponds to the general formula : 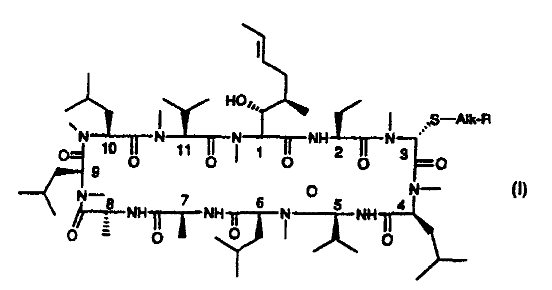 in which:
- Alk represents an alkylene radical containing 2 to 6 carbon atoms in straight or branched chains or a cycloalkylene radical containing 3 to 6 carbon atoms and
- R represents
• either a carboxyl or alkyloxycarbonyl radical,
• or an -NR₁R₂ radical in which R₁ and R₂, which are identical or different, represent hydrogen atoms or alkyl, alkenyl (2 to 4C), cycloalkyl (3 to 6C) or optionally substituted (by a halogen atom, alkyloxy, alkyloxycarbonyl, amino, alkylamino or dialkylamino) phenyl radicals or represent benzyl or heterocyclyl radicals, the heterocyclyl radical being saturated or unsaturated and containing 5 or 6 ring members and 1 to 3 heteroatoms, or in which R₁ and R₂ form, with the nitrogen atom to which they are attached, a saturated or unsaturated heterocycle containing 4 to 6 ring members, which can contain another heteroatom chosen from nitrogen, oxygen or sulphur, optionally substituted by alkyl, phenyl or benzyl,
• or a radical of general formula: 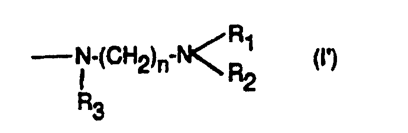 in which R₁ and R₂ are defined as above, R₃ represents a hydrogen atom or an alkyl radical and n is an integer from 2 to 4,
the alkyl portions or radicals defined above being straight or branched and containing 1 to 4 carbon atoms, and its pharmaceutically acceptable salts, when they exist.

2. Cyclosporin derivative according to Claim 1, **characterized in that**:
- Alk represents an alkylene radical containing 2 to 6 carbon atoms in straight or branched chains and
- R represents an -NR₁R₂ radical in which R₁ and R₂, which are identical or different, represent hydrogen atoms or alkyl, alkenyl (2 to 4C) or optionally substituted (by a halogen atom, alkyloxy, alkyloxycarbonyl, amino, alkylamino or dialkylamino) phenyl radicals or represent benzyl radicals or in which R₁ and R₂ form, with the nitrogen atom to which they are attached, a saturated or unsaturated heterocycle containing 4 to 6 ring members, which can contain another heteroatom chosen from nitrogen, oxygen or sulphur, optionally substituted by alkyl, the alkyl portions or radicals defined above being straight or branched and containing 1 to 4 carbon atoms, and its pharmaceutically acceptable salts, when they exist.

3. Cyclosporin derivative according to Claim 1, **characterized in that**:
- Alk represents an alkylene radical containing 2 to 5 carbon atoms in straight or branched chains and
- R represents an -NR₁R₂ radical in which R₁ and R₂, which are identical or different, represent hydrogen atoms or alkyl, allyl, phenyl or benzyl radicals or in which R₁ and R₂ form, with the nitrogen atom to which they are attached, a heterocycle chosen from azetidinyl, piperidyl, piperazinyl, N-methylpiperazinyl, N-phenylpiperazinyl, N-benzylpiperazinyl, imidazolyl, morpholino, tetrahydropyridyl, methyltetrahydropyridyl or phenyltetrahydropyridyl, the alkyl portions or radicals defined above being straight or branched and containing 1 to 4 carbon atoms, and its pharmaceutically acceptable salts, when they exist.

4. Cyclosporin derivative according to Claim 1, **characterized in that** it is [(R)-2-(N,N-diethylamino) ethylthio-Sar]³-cyclosporin A, and its pharmaceutically acceptable salts.

5. Cyclosporin derivative according to Claim 1, **characterized in that** it is [(R)-2-(N,N-dimethylamino)ethylthio-Sar]³-cyclosporin A, and its pharmaceutically acceptable salts.

6. Cyclosporin derivative according to Claim 1, **characterized in that** it is [(R)-2-(1-piperidyl)ethylthio-Sar]³-cyclosporin A, and its pharmaceutically acceptable salts.

7. Cyclosporin derivative according to Claim 1, **characterized in that** it is [(R)-2-(N-methyl-N-i-propylamino)ethylthio-Sar]³-cyclosporin A, and its pharmaceutically acceptable salts.

8. Cyclosporin derivative according to Claim 1,
**characterized in that** it is [(R)-2-(N-methyl-N-t-butylamino)ethylthio-Sar]³-cyclosporin A, and its pharmaceutically acceptable salts.

9. Process for the preparation of Cyclosporin derivative according to Claim 1, **characterized in that** a disulphide of general formula:
R-Alk-S-S-Alk-R
in which R and Alk are defined as above, the functional groups of which can interfere with the reaction have, if appropriate, been protected beforehand, is reacted with an activated form of cyclosporin A, then, if appropriate, the protective radicals are removed and/or the product obtained is optionally converted into a salt, when they exist.

10. Pharmaceutical composition, **characterized in that** it contains at least one product according to Claim 1, in the pure state or in combination with any compatible and pharmaceutically acceptable diluent or adjuvant and/or in combination with another antiviral, immunomodulating or antimicrobial active principle.

11. Synergizing combinations, **characterized in that** they comprise at least one cyclosporin derivative according to Claim 1 and at least one other agent known for its anti-retrovirus activity.
